# EUROPÄISCHE PATENTSCHRIFT

(11) **EP 2 238 436 B1**
(45) Veröffentlichungstag und Bekanntmachung des Hinweises auf die Patenterteilung: **27.04.2011**
(21) Anmeldenummer: 09705391.2
(22) Anmeldetag: 07.01.2009
(51) Int. Cl.: G01N 27/414

(54) **SENSORELEMENT EINES GASSENSORS**
SENSOR ELEMENT OF A GAS SENSOR
ÉLÉMENT DE SONDE POUR SONDE DE GAZ

(30) Priorität: 28.01.2008 DE 102008006326
(43) Veröffentlichungstag der Anmeldung: 13.10.2010
(73) Patentinhaber: Robert Bosch GmbH, 70442 Stuttgart (DE)
(72) Erfinder: WIDENMEYER, Markus, 71101 Schoenaich (DE); ELBE, Dieter, 74343 Sachsenheim (DE)
(86) Internationale Anmeldenummer: PCT/EP2009/050103
(87) Internationale Veröffentlichungsnummer: WO 2009/095285

(56) Entgegenhaltungen:
- EP-A- 0 661 535
- DE-A1-102005 010 032
- DE-A1-102005 010 454
- WIDENMEYER M ET AL: "TiOx overlayers on MCM-48 silica by consecutive grafting" MICROPOROUS AND MESOPOROUS MATERIALS, ELSEVIER SCIENCE PUBLISHING, NEW YORK, US, Bd. 44-45, 6. April 2001 (2001-04-06), Seiten 327-336, XP004247164 ISSN: 1387-1811

## Beschreibung

### Stand der Technik

Die Erfindung bezieht sich auf ein Sensorelement eines Gassensors zur Bestimmung von Gaskomponenten in Gasgemischen, insbesondere in Abgasen von Verbrennungsmotoren, auf ein Verfahren zur Herstellung desselben, sowie auf dessen Verwendung nach dem Oberbegriff der unabhängigen Ansprüche.

Zur Bestimmung von Gaskomponenten in Gasgemischen werden unter anderem Feldeffekttransistoren eingesetzt. Dabei reagiert beispielsweise das Gate bzw. die Gateelektrode des Feldeffekttransistors sensitiv auf zu bestimmende Gaskomponenten, wodurch es zu einer Veränderung einer an der Gateelektrode anliegenden Steuerspannung kommt. Die dabei auftretende Veränderung des zwischen Source- und Drainelektrode des Feldeffekttransistors resultierenden Stromflusses wird detektiert und einer Konzentration der zu bestimmenden Gaskomponente zugeordnet.

Ein derartiger Gassensor ist beispielsweise der DE 10 2005 010 454 A1 zu entnehmen. Dabei weist die Gate-Elektrode eine saure bzw. basische Beschichtung auf, die die Sensitivität des Gassensors auf brandrelevante Gase erhöht. Nachteilig ist daran, dass nur pH-aktive Gase erfasst werden können. Eine Bestimmung von Abgasbestandteilen ohne nennenswerte Querempfindlichkeit auf Kohlenwasserstoffe ist nicht möglich.

Die Selektivität bzw. die Sensitivität eines solchen als Feldeffekttransistor ausgeführten Sensorelementes ist das komplexe Resultat mehrerer Faktoren wie beispielsweise der Zusammensetzung von Einzelkomponenten der Gateelektrode. Dazu zählt bspw. eine als Edelmetallbeschichtung ausgeführte Gatemetallisierung oder eine zwischen Gatemetallisierung und Halbleitersubstrat vorgesehene Isolationsschicht.

### Offenbarung der Erfindung

Der vorliegenden Erfindung liegt die Aufgabe zugrunde, ein Sensorelement zur Bestimmung von Gaskomponenten in Gasgemischen bereitzustellen, das einen Feldeffekttransistor umfasst, der auf Grund einer geeigneten Grenzschicht im Bereich der Gateelektrode besonders sensitiv auf zu bestimmende Gaskomponenten ist.

Ein Sensorelement bzw. ein mittels dem beanspruchten Verfahren hergestelltes Sensorelement mit den kennzeichnenden Merkmalen der unabhängigen Ansprüche löst in vorteilhafter Weise die der Erfindung zugrundeliegende Aufgabe. Dabei weist ein in das Sensorelement integrierter Feldeffekttransistor eine Gateelektrode mit einer Gatemetallisierung auf, die über eine Grenzschicht mit einer Isolationsschicht oder einem Halbleitersubstrat des Feldeffekttransistors in Kontakt steht, wobei die Grenzschicht durch Oberflächenmodifizierung der Isolationsschicht oder des Halbleitersubstrats auf chemischem Wege erzeugt wird, und zwar durch eine Behandlung mit Metallalkoxiden, Metallamiden, Metallhalogeniden oder Metallalkylen.

Weitere vorteilhafte Ausführungsformen der vorliegenden Erfindung ergeben sich aus den Unteransprüchen.

So ist es von Vorteil, wenn die Isolationsschicht aus Siliziumnitrid ausgeführt ist. Dieses weist stets in begrenztem Umfang freie Si-OH-Gruppen auf, die einer chemischen Modifizierung zugänglich sind. Sie reagieren während der Modifizierung mit den eingesetzten Metallalkoxiden, Metallamiden, Metallhalogeniden bzw. Metallalkylen.

Weiterhin ist von Vorteil, wenn die Oberflächenmodifizierung mittels Aufbringung eines Titan- oder Germaniumalkoxides erfolgt. Die genannten Alkoxide sind einfach zu hydrolisieren und bilden bei einer nachfolgenden Wärmebehandlung einen stabilen und chemisch inerten Schichtverbund aus. Gleiches gilt bei der Verwendung von Dialkylamiden bzw. Dimethylsilylamiden des Titans bzw. Bismuts. Auch bei Verwendung von Tetraalkylgermaniumverbindungen ist eine einfach durchzuführende Oberflächenmodifizierung zur Erzeugung einer Grenzschicht zu erwarten.

Darüber hinaus ist von Vorteil, wenn bei Verwendung von Metallamiden zur Erzeugung der Grenzschicht nach der Hydrolyse und Dehydratisierung der aufgebrachten Metallamide eine Behandlung mit einer Mischung einer Mineralsäure in einem Alkohol erfolgt. Auf diese Weise können noch in der Oberflächenbeschichtung enthaltene Amine erfolgreich herausgelöst werden.

Das beschriebene Sensorelement lässt sich in vorteilhafter Weise einsetzen zur Bestimmung von Gaskomponenten in Abgasen von Verbrennungsmotoren, Kraftwerksanlagen oder Heizgeräten. Es eignet sich weiterhin in vorteilhafter Weise zur Überprüfung der Funktionstüchtigkeit eines NOX-Speicherkatalysators oder eines SCR-Abgasnachbehandlungssystems.

### Kurze Beschreibung der Zeichnung

Ein Ausführungsbeispiel der Erfindung ist in der Zeichnung dargestellt und in der nachfolgenden Beschreibung näher erläutert. So zeigt:
- Figur 1: eine schematische Schnittdarstellung eines Sensorelementes gemäß einer ersten Ausführungsform der vorliegenden Erfindung und
- Figur 2: eine schematische Abfolge der bei einer chemischen Modifizierung des Gatematerials vorgesehenen Verfahrensschritte.

### Ausführungsformen der Erfindung

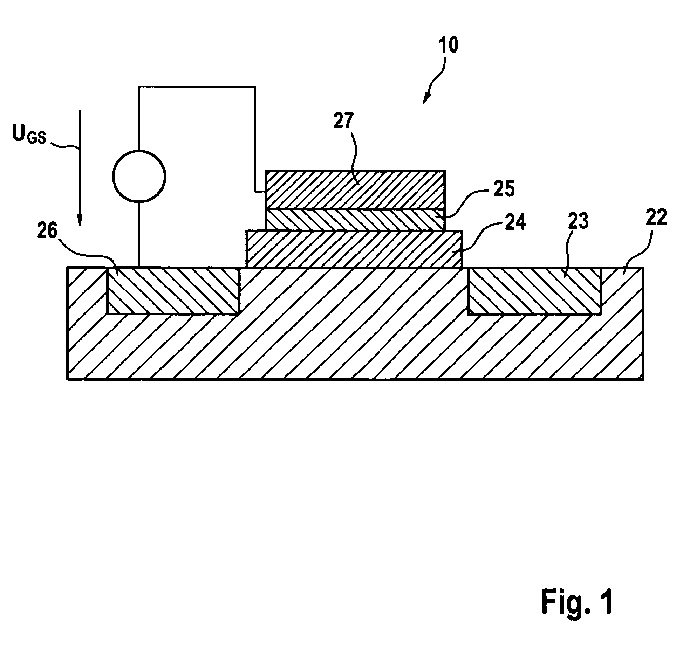

In Figur 1 ist ein Sensorelement gemäß einem ersten Ausführungsbeispiel der vorliegenden Erfindung dargestellt. Das Sensorelement 10 ist als Feldeffekttransistor (FET) bzw. als chemisch sensitiver Feldeffekttransistor (CHEMFET) ausgeführt. Das Sensorelement 10 in Form eines Feldeffekttransistors umfasst ein Halbleitersubstrat 22, welches beispielsweise aus Galliumnitrid, Aluminiumnitrid, Galliumaluminiumnitrid oder Siliziumcarbid ausgeführt ist. Diese Materialien sind jeweils geeignet dotiert oder enthalten bspw. im Fall von Galliumnitrid eine wenige Nanometer dicke Schicht aus Galliumaluminiumnitrid. Das Halbleitersubstrat 22 ist mit dem Kontakt 26 einer Sourceelektrode und dem Kontakt 23 einer Drainelektrode versehen. Weiterhin umfasst das Sensorelement 10 eine Gatemetallisierung 27, die über eine bspw. aus Siliciumnitrid ausgeführte Isolationsschicht 24 mit dem Halbleitermaterial 22 in physischem Kontakt steht. Dabei dient die Isolationsschicht 24 der Verhinderung von Gate-Leckströmen und einer möglichen Elektromigration. Auf diese Weise wird der elektrische Betrieb sichergestellt und eine einfache Signalauswertung ermöglicht.

Ist die Gatemetallisierung 27 in geeigneter Weise sensitiv für zu vermessende Gaskomponenten ausgeführt, so verändert sich eine, zwischen dem Kontakt 26 der Sourceelektrode und der Gatemetallisierung 27 anliegende Spannung U_{GS} in ihrer Höhe in Abhängigkeit von der Konzentration der zu bestimmenden Gaskomponenten.

Weiterhin kann erfindungsgemäß die Sensitivität der aus Halbleitersubstrat 22, Isolationsschicht 24 und Gatemetallisierung 27 gebildeten Gateleektrode dadurch erhöht werden, dass zwischen der Isolationsschicht 24 und der Gatemetallisierung 27 eine Grenzschicht 25 vorgesehen ist. Gemäß einer alternativen Ausführungsform ist es auch möglich, auf eine separate Isolationsschicht 24 zu verzichten. In diesem Fall grenzt die Grenzschicht 25 auf der einen Seite an das Material des Halbleitersubstrates 22 an und auf der gegenüberliegenden Seite an die Gatemetallisierung 27.

Erfindungsgemäß wird die Grenzschicht 25 erzeugt, indem die Isolationsschicht 24 bzw. das Halbleitersubstrat 22 insbesondere im Bereich des Gates in geeigneter Weise mit Metallalkoxiden, Metallamiden, Metallhalogeniden und/ oder Metallalkylen behandelt wird. Bei Behandlung der Oberfläche der Isolationsschicht 24 bzw. des Halbleitersubstrats 22 mit den genannten Metallverbindungen kommt es zu einer Reaktion oberflächennaher Hydroxidgruppen des Materials der Isolationsschicht 24 bzw. des Halbleitersubstrats 22 mit den genannten Metallverbindungen. Weist die Oberfläche der Isolationsschicht 24 bzw. des Halbleitersubstrats 22 eine zu geringe Dichte an oberflächennahen Hydroxidgruppen auf, so kann durch oxidaktive oder hydrothermale Verfahren deren Anzahl gezielt erhöht werden. Weiterhin können anstelle der erwähnten Alkoxide die reaktiveren Metallalkyle bzw. -amide gegebenenfalls bei höherer Temperatur eingesetzt werden.

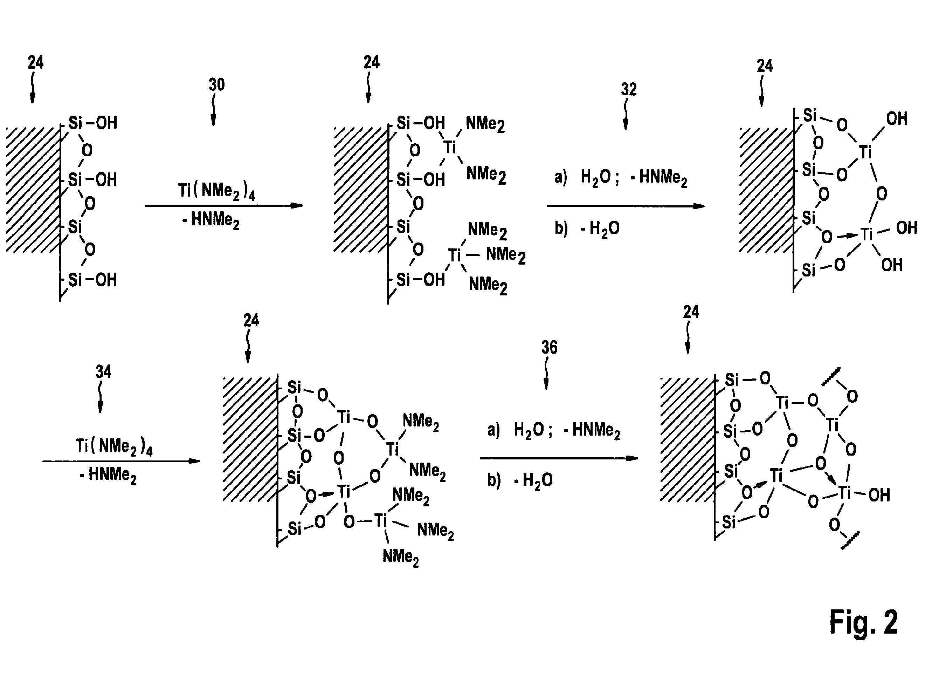

Das dem Schichtaufbau zur Erzeugung der Grenzschicht 25 zugrundeliegende Verfahren ist in Figur 2 verdeutlicht. Exemplarisch wird dabei die Isolationsschicht 24 mit einer Grenzschicht 25 versehen, in gleicher Weise kann jedoch auch das Halbleitersubstrat 22 des Feldeffekttransistors mit einer entsprechenden Grenzschicht 25 versehen werden.

Zur Erzeugung einer Grenzschicht 25 auf einer Großfläche der Isolierschicht 24 des Feldeffekttransistors bzw. auf dessen Halbleitersubstrat 22 wird deren Oberfläche mit einem Metallalkoxid, einem Metallamid, einem Metallhalogenid und/ oder einem Metallalkyl behandelt. Dabei reagieren die genannten Metallverbindungen mit freien Hydroxidgruppen auf der Oberfläche der Isolierschicht 24 bzw. des Halbleitersubstrates 22. Die bei dieser Reaktion ablaufenden Vorgänge sind in Figur 2 exemplarisch für die Reaktion von Titantetra(dialkylamid) in Form eines ersten Prozessschritts 30 verdeutlicht.

Sollte die Zahl der auf der Oberfläche der Isolierschicht 24 bzw. des Halbleitersubstrats 22 vorhandenen Hydroxidgruppen nicht ausreichend sein, so kann der Behandlung mit einer der genannten Metallverbindungen eine Vorbehandlung beispielsweise in Form einer Hochtemperaturbehandlung an Luft oder in einer Wasserdampfatmosphäre vorangestellt werden.

Die mittels der genannten Metallverbindungen modifizierte Oberfläche der Isolierschicht 24 bzw. des Halbleitersubstrats 22 wird beispielsweise in einem weiteren Prozessschritt 32 zunächst hydrolisiert, woran sich eine Dehydratisierungsreaktion anschließt. Es resultiert ein hydroxidgruppenhaltiges Metalloxidnetzwerk. Dabei ist dieses Reaktionsschema grundsätzlich beliebig oft durchführbar, wobei durch Wiederholung der Prozessschritte 30, 32 die Schichtdicke der entstehenden Grenzschicht 25 gezielt eingestellt werden kann.

Als Metallverbindung sind beispielsweise Metallalkoxide vorzugsweise in Form von Titanalkoxiden wie Titan(diisopropyloxid)dichlorid oder Titantetrakis-(isopropyloxid) sowie Germaniumalkoxide wie bspw. Tetraethoxigermanium geeignet. Weiterhin sind als Metallverbindungen Metallamide wie beispielsweise Titanbisdialkylamide oder Titantetradialkylamide wie bspw. Titantetrakis(diethylamid) oder Titantetrakis(dimethylamid) geeignet sowie Titanbis(dimethylsilylamide), Titantetra(dimethylsilylamide) oder Bismutbis(trimethylsilylamide). Letztlich sind als Metallverbindungen auch Metallalkyle wie beispielsweise Tetraalkylgermaniumverbindungen geeignet.

Das in Figur 2 verdeutlichte Verfahren kann beispielsweise gemäß folgendem Ausführungsbeispiel durchgeführt werden.

Die aus Siliziumnitrid bestehende Oberfläche der Isolationsschicht 24 wird in eine Lösung von Titantetrakis(dimethylamid) in Hexan mit einer Konzentration von einem Millimol pro Liter getaucht und nachfolgend mit Hexan gespült. Anschließend wird der Feldeffekttransistor bei ca. 400°C an Luft für eine Stunde erwärmt. Dieser Gesamtprozess wird insgesamt dreimal wiederholt. Es resultiert eine Grenzschicht 25, die durch eine lateral regelmäßige mono- bis oligomolekulare Schicht von Fremdelementoxiden bezogen auf das Grundmaterial der Isolationsschicht 24 gebildet ist. Abschließend erfolgt die Aufbringung einer Edelmetallmetallisierung als Gatemetallisierung 27 auf die erzeugte Grenzschicht 25.

Da beim Einsatz von Metallamiden oftmals bei einer nachfolgenden Hydrolyse zunächst nicht alle entstehenden Amine restlos von der zur Ausbildung der Grenzschicht 25 behandelten Oberfläche entfernt werden, kann beispielsweise der Feldeffekttransistor nachfolgend mit einer Mischung von Ethanol und Salzsäure im Verhältnis 100 : 1 behandelt werden.

Das auf diese Weise erzeugte Sensorelement 10 eignet sich insbesondere zur Bestimmung von Gaskomponenten in Gasgemischen, wie beispielsweise in Abgasen von Verbrennungsmotoren, Heizungsanlagen und in Kraftwerksanwendungen. Dabei ist das Sensorelement insbesondere geeignet zur Detektion von Stickoxiden in Verbrennungsabgasen, beispielsweise zu Zwecken der On-Board-Diagnose bei Kraftfahrzeugen oder zur Überwachung von Abgasreinigungssystemen wie beispielsweise Stickoxidspeicherkatalysatoren oder SCR-Systemen.

## Patentansprüche

1. Sensorelement eines Gassensors zur Bestimmung von Gaskomponenten in Gasgemischen, welches einen Feldeffekttransistor mit einer Source- und einer Drainelektrode sowie einer Gateelektrode enthält, wobei die Gateelektrode eine Gatemetallisierung (27) umfasst, **dadurch gekennzeichnet, dass** die Gatemetallisierung (27) über eine Grenzschicht (25) mit einer Isolationsschicht (24) oder einem Halbleitersubstrat (22) des Feldeffekttransistors in Kontakt steht, wobei die Grenzschicht (25) durch Oberflächenmodifizierung der Isolationsschicht (24) oder des Halbleitersubstrats (22) mit Metallalkoxiden, Metallamiden, Metallhalogeniden und/oder Metallalkylen gebildet ist.

2. Sensorelement nach Anspruch 1, **dadurch gekennzeichnet, dass** die Isolationsschicht (24) aus Siliciumnitrid ausgeführt ist.

3. Sensorelement nach Anspruch 1 oder 2, **dadurch gekennzeichnet, dass** das Metallalkoxid ein Titanalkoxid oder ein Germaniumalkoxid ist.

4. Sensorelement nach einem der Ansprüche 1 bis 3, **dadurch gekennzeichnet, dass** das Metallamid ein Titan(dialkylamid), ein Titantetradialkylamid, ein Titan(bis(dimethylsilyl)amid oder ein Bismuth(bis(trimethylsilyl)amid) ist.

5. Sensorelement nach einem der vorhergehenden Ansprüche, **dadurch gekennzeichnet, dass** das Metallalkyl eine Tetraalkylgermaniumverbidnung ist.

6. Verfahren zur Herstellung eines Sensorelementes für Gassensoren zur Bestimmung von Gaskomponenten in Gasgemischen, insbesondere zur Herstellung eines Sensorelementes nach einem der Ansprüche 1 bis 5, wobei in eine Gateelektrode eines Feldeffekttransistors eine Grenzschicht integriert wird, **dadurch gekennzeichnet, dass** die Oberfläche einer Isolationsschicht (24) oder eines Halbleitersubstrats (22) zur Ausbildung der Grenzschicht (25) mit einem Metallalkoxid, einem Metallamid, einem Metallhalogenid und/oder einem Metallalkyl behandelt wird.

7. Verfahren nach Anspruch 6, **dadurch gekennzeichnet, dass** nach der Behandlung mit einem Metallalkoxid, Metallamid, Metallhalogenid und/oder Metallalkyl in einem zweiten Schritt eine Hydrolyse und nachfolgend eine Dehydratisierung des aufgebrachten Metallalkoxids, Metallamids, Metallhalogenids und/oder Metallalkyls erfolgt.

8. Verfahren nach Anspruch 6 oder 7, **dadurch gekennzeichnet, dass** in einem dritten Schritt eine Behandlung mit einer Mischung einer Mineralsäure in einem Alkohol erfolgt.

9. Verwendung eines Sensorelementes nach einem der Ansprüche 1 bis 5 zur Bestimmung von Gaskomponenten in Abgasen von Verbrennungsmotoren, Kraftwerksanlagen oder Heizgeräten.

10. Verwendung eines Sensorelementes nach einem der Ansprüche 1 bis 5 zur Überwachung der Funktionstüchtigkeit eines NOx-Speicherkatalysators oder eines SCR-Abgasnachbehandlungssystems.

## Claims

1. Sensor element of a gas sensor for determining gas components in gas mixtures, which contains a field effect transistor having a source electrode and a drain electrode and also a gate electrode, the gate electrode comprising a gate metallization (27), **characterized in that** the gate metallization (27) is in contact with an insulation layer (24) or a semiconductor substrate (22) of the field effect transistor via a boundary layer (25), the boundary layer (25) being formed by surface modification of the insulation layer (24) or of the semiconductor substrate (22) with metal alkoxides, metal amides, metal halides and/or metal alkyls.

2. Sensor element according to Claim 1, **characterized in that** the insulation layer (24) is made of silicon nitride.

3. Sensor element according to Claim 1 or 2, **characterized in that** the metal alkoxide is a titanium alkoxide or a germanium alkoxide.

4. Sensor element according to any of Claims 1 to 3, **characterized in that** the metal amide is a titanium (dialkylamide), a titanium tetradialkylamide, a titanium (bis(dimethylsilyl)amide) or a bismuth (bis(trimethylsilyl)amide).

5. Sensor element according to one of the preceding claims, **characterized in that** the metal alkyl is a tetraalkyl germanium compound.

6. Method for producing a sensor element for gas sensors for determining gas components in gas mixtures, in particular for producing a sensor element according to any of Claims 1 to 5, a boundary layer being integrated into a gate electrode of a field effect transistor, **characterized in that** the surface of an insulation layer (24) or of a semiconductor substrate (22) is treated with a metal alkoxide, a metal amide, a metal halide and/or a metal alkyl in order to form the boundary layer (25).

7. Method according to Claim 6, **characterized in that**, after the treatment with a metal alkoxide, metal amide, metal halide and/or metal alkyl, in a second step, a hydrolysis is effected and, subsequently, a dehydration of the applied metal oxide, metal amide, metal halide and/or metal alkyl is effected.

8. Method according to Claim 6 or 7, **characterized in that** a treatment with a mixture of a mineral acid in an alcohol is effected in a third step.

9. Use of a sensor element according to any of Claims 1 to 5 for determining gas components in exhaust gases of internal combustion engines, power plants or heating devices.

10. Use of a sensor element according to any of Claims 1 to 5 for monitoring the functionality of an NOx storage catalyst or of an SCR exhaust-gas aftertreatment system.

## Revendications

1. Elément de détection d'un détecteur de gaz destiné à déterminer la présence de composants gazeux dans des mélanges de gaz et qui contient un transistor à effet de champ doté d'une électrode de source, d'une électrode de drain ainsi que d'une électrique de grille, l'électrode de grille comprenant une métallisation de grille (27),
**caractérisé en ce que**
la métallisation de grille (27) est en contact par l'intermédiaire d'une couche frontière (25) avec une couche isolante (24) ou un substrat semi-conducteur (22) du transistor à effet de champ, la couche frontière (25) étant formée par modification de la surface de la couche isolante (24) ou du substrat semi-conducteur (22) à l'aide d'alcoxydes de métal, d'amides de métal, d'halogénures de métal et/ou d'alkyles de métal.

2. Elément de détection selon la revendication 1, **caractérisé en ce que** la couche isolante (24) est réalisée en nitrure de silicium.

3. Elément de détection selon la revendication 1 ou 2, **caractérisé en ce que** l'alcoxyde de métal est un alcoxyde de titane ou un alcoxyde de germanium.

4. Elément de détection selon l'une des revendications 1 à 3, **caractérisé en ce que** l'amide de métal est un (dialkylamide) de titane, un tétradialkylamide de titane, un bis(diméthylsilyl)amide de titane ou un bis(triméthylsilyl)amide de bismuth.

5. Elément de détection selon l'une des revendications précédentes, **caractérisé en ce que** l'alkyle de métal est un composé tétraalkylé de germanium.

6. Procédé de fabrication d'un élément de détection pour des détecteurs de gaz destinés à déterminer la présence de composants gazeux dans des mélanges de gaz, en particulier procédé de fabrication d'un élément de détection selon l'une des revendications 1 à 5, dans lequel une couche frontière est intégrée dans une électrode de grille d'un transistor à effet de champ,
**caractérisé en ce que**
la surface d'une couche isolante (24) ou d'un substrat semi-conducteur (22) est traitée à l'aide d'un alcoxyde de métal, d'un amide de métal, d'un halogénure de métal et/ou d'un alkyle de métal pour former la couche frontière (25).

7. Procédé selon la revendication 6, **caractérisé en ce qu'**après le traitement à l'aide d'un alcoxyde de métal, d'un amide de métal, d'un halogénure de métal et/ou d'un alkyle de métal, dans une deuxième étape, une hydrolyse suivie d'une déshydratation de l'alcoxyde de métal, de l'amide de métal, de l'halogénure de métal et/ou de l'alkyle de métal appliqués sont réalisées.

8. Procédé selon la revendication 6 ou 7, **caractérisé en ce que** dans une troisième étape, un traitement à l'aide d'un mélange d'un acide minéral ou d'un alcool est réalisé.

9. Utilisation d'un élément de détection selon l'une des revendications 1 à 5 pour la détermination de la présence de composants gazeux dans les gaz d'échappement de moteurs à combustion interne, d'installations de centrale électrique ou d'appareils de chauffage.

10. Utilisation d'un élément de détection selon l'une des revendications 1 à 5 pour la surveillance du bon fonctionnement d'un catalyseur-accumulateur de NOx ou d'un système de traitement des gaz d'échappement par SCR.
